# EUROPÄISCHE PATENTSCHRIFT

(11) **EP 1 479 781 B1**
(45) Veröffentlichungstag und Bekanntmachung des Hinweises auf die Patenterteilung: **05.11.2008**
(21) Anmeldenummer: 04011220.3
(22) Anmeldetag: 12.05.2004
(51) Int. Cl.: C12Q 1/68

(54) **Verfahren zum Nachweis von Nukleinsäuren**
Method for detecting nucleic acids
Méthode de détection d'acides nucléiques

(30) Priorität: 21.05.2003 DE 10322912
(43) Veröffentlichungstag der Anmeldung: 24.11.2004
(73) Patentinhaber: Bayer Technology Services GmbH, 51368 Leverkusen (DE)
(72) Erfinder: Burmeister, Jens Dr., 51061 Köln (DE); Diessel, Edgar Dr., 51061 Köln (DE)
(74) Vertreter: Thomaier, Jörg

(56) Entgegenhaltungen:
- WO-A-00/25136
- WO-A-02/02810
- WO-A-99/57550
- WO-A-03/023061
- ANDREW TATON T ET AL: "Scanometric DNA Array Detection with Nanoparticle Probes" SCIENCE, AAAS. LANCASTER, PA, US, Bd. 289, 8. August 2000 (2000-08-08), Seiten 1757-1760, XP002158394 ISSN: 0036-8075
- PARK S-J ET AL: "Array-based electrical detection of DNA with nanoparticles probes" SCIENCE, AMERICAN ASSOCIATION FOR THE ADVANCEMENT OF SCIENCE, US, Bd. 295, 22. Februar 2002 (2002-02-22), Seiten 1503-1506, XP001160895 ISSN: 0036-8075
- GONZALEZ M ET AL: "Extremely high thermal stability of streptavidin and avidin upon biotin binding" BIOMOLECULAR ENGINEERING, ELSEVIER, NEW YORK, NY, US, Bd. 16, Nr. 1-4, 31. Dezember 1999 (1999-12-31), Seiten 67-72, XP004257800 ISSN: 1389-0344
- BURMEISTER JENS ET AL: "Single nucleotide polymorphism analysis by chip-based hybridization and direct current electrical detection of gold-labeled DNA." ANALYTICAL AND BIOANALYTICAL CHEMISTRY. JUN 2004, Bd. 379, Nr. 3, Juni 2004 (2004-06), Seiten 391-398, XP008033606 ISSN: 1618-2642

## Beschreibung

Die Erfindung betrifft eine Methode zum spezifischen Nachweis von Nukleinsäuren an einer festen Phase. Die Erfindung betrifft außerdem Kits, die diejenigen Reagenzien enthalten, die zur Durchführung der beschriebenen Assays benötigt werden. Der Nachweis von Desoxyribonukleinsäuren (DNA) oder Ribonukleinsäuren (RNA) hat ein großes Anwendungsgebiet z.B. in der Human- oder Veterinärdiagnostik, im Nahrungsmittelbereich, in der Umweltanalytik, im Pflanzenschutz, in der biochemischen bzw. pharmakologischen Forschung sowie in der forensischen Medizin.

Als eine Standardmethode zum Nachweis von Nukleinsäuren haben sich DNA-Arrays bewährt, die die gleichzeitige Analyse einer großen Zahl verschiedener Sequenzen ermöglichen. DNA-Arrays finden z.B. Verwendung für die Expressions- Profilierung, Sequenzierung durch Hybridisierung, Analyse von Einzelnukleotid-Polymorphismen (SNP) usw. Beispiele zur Herstellung und Verwendung von DNA-Arrays finden sich z.B. in DNA Microarrays, D. Bowell and J. Sambrook (eds.), Cold Spring Harbor Laboratory Press, New York 2003.

Als Detektionselemente auf DNA Arrays können Nukleinsäuren oder Nukleinsäure-Analoga verwendet werden. Beispiele für Nukleinsäure-Analoga sind PNA (M. Egholm et al. Nature 365, 566-568 (1993)), LNA (D.A. Braasch, D.R. Corey, Chem. Biol. 8, 1-7, (2001), oder am Zuckergerüst modifizierte Nukleinsäuren (M. Shimizu et al., FEBS Letters 302, 155-158 (1992)).

DNA-Arrays können z.B. durch optische, elektrische, mechanische oder magnetische Verfahren ausgelesen werden.

Optische Detektionsverfahren basieren z.B. auf dem Nachweis fluoreszenzmarkierter Biomoleküle an dielektrischen Oberflächen. Die Fluoreszenzanregung kann hierbei über planare optische Wellenleiter (siehe US-A-5,959,292), Totalreflexion an Grenzflächen (siehe DE 196 28 002 A2), oder an der Oberfläche von Lichtleitfasern erfolgen (siehe US-A- 4,447,546).

Elektrische Biosensoren beruhen z.B. auf dem Nachweis von Analyten, die durch metallische Partikel, z.B. Nanopartikel markiert sind. Zur Detektion werden diese Partikel durch autometallographische Abscheidung soweit vergrößert, dass sie einen mikrostrukturierten Stromkreis kurzschließen. Dies wird durch eine einfache Gleichstrom-Widerstandsmessung demonstriert (US-A-4,794,089; US-A-5,137,827; US-A-5,284,748).

Feldeffekttransistoren können als elektronische Transducer z.B. für eine enzymatische Reaktion eingesetzt werden: Zayats et al. Biosens. & Bioelectron. 15, 671 (2000).

Als mechanische Transducer werden Schwingquarze beschrieben, bei denen die Veränderung der Resonanzfrequenz durch Massenbelegung erfolgt: Steinem et al. Biosens. & Bioelectronics 12, 787 (1997). In einem alternativen mechanischen Transducer werden mit Interdigitalstrukturen Oberflächenwellen angeregt, die durch Targetadsorption modifiziert werden: Howe et al., Biosens. & Bioelectron. 15, 641 (2000).

Werden die Targetmoleküle mit magnetischen Beads markiert, so kann die Erkennungsreaktion über den magnetischen Einfluss der Beads auf den Giant Magnetic Resistance (GMR) eines entsprechenden Widerstands detektiert werden: Baselt et al. Biosens. and Bioelectron. 13, 731 (1998).

Insbesondere für das elektrische Verfahren ist eine Markierung der an den Array hybridisierten Target-Nukleinsäuren mit elektrisch leitfähigen Partikeln vorteilhaft. WO 99/57550-A2 beschreibt die Markierung von Nukleinsäure-Targets, die an immobilisierte Nukleinsäuren zwischen planaren Elektrodenpaaren hybridisiert wurden, mit z.B. Goldpartikeln. Durch autometallographische Verstärkung der Goldpartikel mit z.B. Lösungen von Metallionen in Anwesenheit von Reduktionsmitteln kann ein elektrisch leitfähiger, metallischer Film, der i. allg. aus einem Netzwerk von miteinander elektrisch leitfähig verbundenen Partikeln besteht, zwischen den Elektrodenpaaren erzeugt werden. Durch Nachweis der Leitfähigkeit des metallischen Films wird die Anwesenheit des Targets nachgewiesen. In WO 99/57550-A2 wird beschrieben, dass die Markierung des Targets vor oder nach der Wechselwirkung mit den immobilisierten Detektorelementen erfolgen kann. In einer bevorzugten Ausführungsform von WO 00/25136-A2 wird die Markierung eines Oligonukleotids mit cis-Platin-Biotin sowie die anschließende Markierung des biotinylierten Targets mit einem Streptavidin-Goldcluster beschrieben. Das Gold-markierte Target wird an immobilisierte Nukleinsäuren zwischen Elektrodenpaaren hybridisiert. Nach autometallographischer Verstärkung mit Goldsalzen in Anwesenheit von Reduktionsmitteln wird ein leitfähiger Goldfilm analog zu dem oben beschriebenen Silberfilm ausgebildet. Die Messung des elektrischen Kontaktes zwischen den Elektrodenpaaren indiziert das Hybridisierungsereignis.

In einer weiteren bevorzugten Ausführungsform von WO 00/25136-A2 wird die Reaktion eines Targetmoleküls mit Biotin beschrieben. Das Biotin-markierte Target wird an zwischen Elektrodenpaaren immobilisierte Detektorelemente gebunden. Anschließend erfolgt die Markierung der gebundenen biotinylierten Tagets mit kolloidalen Goldpartikeln, die mit Avidin oder Streptavidin verknüpft wurden. Nach autometallographischer Verstärkung mit Goldsalzen in Anwesenheit von Reduktionsmitteln wird ein leitfähiger Goldfilm ausgebildet. Die Messung des elektrischen Kontaktes zwischen den Elektrodenpaaren indiziert das Bindungsereignis.

Die Detektion von Nukleinsäuren, die an immobilisierte Detektorelemente hybridisiert und mit Goldpartikeln markiert wurden, kann auch durch optische Verfahren erfolgen. WO 02/02810-A2 beschreibt die Ausbildung von Niederschlägen auf Array-Elementen, sowie die Ermittlung des zeitlichen Verlaufs der Niederschlagsbildung in Form von Signalintensitäten durch z.B. optische oder elektrische Verfahren. WO 02/02810-A2 beansprucht, dass die Verknüpfung der Targets mit z.B. kolloidalen Metallpartikeln vor, während oder nach der Wechselwirkung mit den immobilisierten Detektorelementen erfolgt.

Zwei verschiedene Methoden zum Nachweis von Nukleinsäuren auf Microarrays basierend auf der Markierung mit Goldpartikeln sind derzeit kommerziell erhältlich. Das ArrayTube System (Clondiag Chip Technologies) beruht auf der Hybridisierung von z.B. mit biotinylierten Primern amplifizierten Targets, die unter stringenten Bedingungen an den Chip hybridisiert werden. Anschließend erfolgt die Markierung der Targets mit Streptavidin-Gold (kolloidales Gold, 5nm). Der optische Nachweis der Hybridisierung erfolgt nach autometallographischer Verstärkung mit Silbersalzen. Das ArrayTube Instruction Manual empfiehlt ausdrücklich, die Gold-Markierung erst nach der stringenten Hybridisierung durchzuführen.

Das ebenfalls kommerziell erhältliche Nachweissystem der Firma Genicon Sciences basiert auf dem optischen Nachweis von Gold-Partikeln, die mit anti-Biotin Antikörpern verknüpft wurden. Eine autometallographische Verstärkung der Goldpartikel ist für die Anwendung der Resonance Light Scattering Technologie nicht erforderlich. In allen von der Firma Genicon Sciences beschriebenen Anwendungen der RLS-Technologie auf Nukleinsäuren findet die Markierung der biotinylierten Targets erst nach der stringenten Hybridisierung an den Array statt.

Zusammenfassend kann festgestellt werden, dass der Nachweis von Nukleinsäuren auf DNA-Microarrays durch Markierung mit Goldpartikeln, die z.B. mit Streptavidin oder Antikörpern verknüpft wurden, gemäß dem derzeitigen Stand der Technik vorzugsweise erst nach der Hybridisierung von Nukleinsäuren an den DNA-Array und nach der Durchführung der Diskriminierung erfolgt.

Ein alternativer Ansatz zur Markierung von Nukleinsäuren auf DNA-Arrays mit Goldpartikeln beruht auf der Verwendung von DNA-beschichteten Goldpartikeln als Markierungseeinheiten. Bei diesem Verfahren wird die nachzuweisende DNA in einem Sandwich-Hybridisierungs-Assay zwischen einer immobilisierten Detektor-DNA, dem Target sowie einem DNA-beschichteten Goldpartikel detektiert. In diesem Verfahren findet keine direkte Anbindung von Gold an die nachzuweisende Nukleinsäure statt; vielmehr wird die Anbindung eines DNA-beschichteten Goldpartikels durch eine zusätzliche Hybridisierung vermittelt. Die Verwendung DNA-beschichteter Goldpartikel zur optischen Detektion von Nukleinsäuren auf DNA-Microarrays wurde z.B. von Taton et al. beschrieben (Taton et al., Science 2000, 289, 1757-1760). Das gleiche Verfahren wurde auch zur elektrischen Detektion von Nukleinsäuren auf DNA-Microarrrays verwendet, beschrieben z.B. in S.-J. Park et al., Science 2002, 295, 1503-1506. Aus den Arbeiten zur Markierung von an DNA-Microarrays hybridisierte Nukleinsäuren mit DNA-beschichteten Goldpartikeln geht hervor, dass diese Partikel die Spezifität der Unterscheidung eng verwandter DNA-Sequenzen erhöhen, indem die Schmelzpunkte der DNA-Sandwich-Komplexe gegenüber nativen DNA-Sandwich-Komplexen angehoben werden. Dieses verbessert insbesondere die Unterscheidung von Einzelnukleotid Polymorphismen (SNPs). Nachteile der Verwendung DNA-beschichteter Goldpartikel liegen in der sehr aufwändigen Herstellung, der Instabilität der Konjugate (werden Goldpartikel z.B. mit thiolierten Oligonukleotiden beschichtet, so sind diese Konjugate instabil gegen Salzlösungen höherer Konzentration, sowie gegen höhere Temperaturen und neigen zur Agglomeration), der hohen Herstellungskosten aufgrund der großen Menge benötigter Oligonukleotide pro Goldpartikel sowie der Abhängigkeit der Markierung von einer spezifischen Hybridisierung.

Letzterer Punkt ist hervorzuheben, da der besondere Vorteil von DNA-Arrays in der gleichzeitigen Analyse einer großen Zahl verschiedener Sequenzen liegt. Werden z.B. verschiedene Gene nebeneinander analysiert, so benötigt man im Falle eines Sandwich-Assays für jedes Gen einen sequenzspezifischen Detektor. Dieser Aufwand schränkt die praktikable Multiplexfähigkeit eines mit DNA-beschichteten Goldpartikeln markierten DNA-Arrays empfindlich ein.

Ein grundsätzliches Problem beim Nachweis von Nukleinsäuren auf DNA-Microarrays ist die gleichzeitige Gewährleistung von Selektivität und Empfindlichkeit der Hybridisierung. Eine besonders hohe Selektivität der Hybridisierung muss dann gewährleistet werden, wenn Einzelnukleotid Polymorphismen (SNPs) z.B. durch Allel-spezifische Hybridisierung auf DNA-Arrays nachgewiesen werden. Ein Beispiel zur SNP-Detektion durch Allel-spezifische Hybridisierung auf DNA-Arrays ist beschrieben in Iwasaka et al., DNA Research 2002, 9, 59-62. Besonders problematisch bei der Allel-spezifischen Hybridisierung ist die Unterscheidung solcher Basenpaarungen zwischen Target und immobilisierter Probe, die sich in ihrer thermodynamischen Stabilität nur wenig unterscheiden. Beispiele für thermodynamisch ähnlich stabile Basenpaarungen sind GC- und GT- oder GC- und GG-Basenpaare.

Die Selektivität der Allel-spezifischen Hybridisierung wird nach dem derzeitigen Stand der Technik entweder durch stringente Hybridisierungsbedingungen oder aber durch stringente Waschschritte nach der ersten, nicht stringenten Hybridisierung erzielt. Die so erreichbare Selektivität bei stringenten Hybridisierungen vermindert jedoch die absoluten Signale der Hybridisierungsreaktion für die "passenden" (in der Regel Watson-Crick) Basenpaare.

Die Verminderung der absoluten Signale infolge stringenter Hybridiserungsbedingungen auf DNA-Arrays ist insbesondere dann von Nachteil, wenn ein DNA-Array mit Gold markiert, anschließend mit Silber verstärkt und elektrisch ausgelesen werden soll, da die Wahrscheinlichkeit der Ausbildung von Perkolationswegen zwischen Gold-Kolloiden von der Oberflächendichte der Kolloide abhängt. Insbesondere muss für ein derartiges Messverfahren eine kritische Oberflächendichte, die einen Schwellwert für die elektrische Leitfähigkeit darstellt, überschritten werden (D. Stauffer, A. Aharony: Perkolationstheorie - Eine Einführung, VCH, Weinheim, 1995, S. 95ff).

Die oben beschriebenen Verfahren zur Hybridisierung von Nukleinsäuren an DNA-Microarrays und Markierung mit Goldpartikeln haben eine Reihe von Nachteilen; insbesondere dann, wenn anschließend eine elektrische Auslesung erfolgen soll. In gemäß dem Stand der Technik ausgeführten Verfahren zur Markierung mit z.B. Streptavidin- oder Antikörper-beschichteten Goldpartikeln wird das Problem der Verringerung der absoluten Signale nach stringenten Hybridisierungen nicht gelöst. Verfahren, die DNA-beschichtete Goldpartikel zur Markierung von DNA-Arrays verwenden, ermöglichen zwar eine gleichzeitig empfindliche und spezifische Detektion eng verwandter Nukleinsäuren, sind jedoch auf Sandwich-basierte Assays beschränkt.

Der Erfindung liegt die Aufgabe zugrunde, die Markierung von DNA-Arrays mit Markierungseinheiten derart zu verbessern, dass die Oberflächendichte der Markierungseinheiten unter Erhalt der Spezifität der Hybridisierung erhöht wird. Die Aufgabe besteht insbesondere darin, die Menge der nachzuweisenden Nukleinsäure-Moleküle, die an den Chip hybridisiert wurden, unter Erhalt der Selektivität der Hybridisierung deutlich zu erhöhen.

Diese Aufgabe wird erfindungsgemäß dadurch gelöst, dass Nukleinsäure-Targets nichtstringent an auf DNA-Arrays immobilisierte Detektor-Nukleinsäuren hybridisiert werden, die Markierung der an die Detektor-Nukleinsäuren hybridisierten Nukleinsäure-Targets mit Markierungseinheiten vor oder nach diesem Hybridisierungschritt erfolgt und anschließend die Diskriminierung erfolgt. Die Diskriminierung verschiedener markierter Sequenzen erfolgt z.B. durch stringente Waschschritte. Überraschenderweise zeigte sich, dass z.B. eine Goldmarkierung zu einer deutlichen Anhebung der Temperatur, die für eine Diskriminierung verschiedener Nukleinsäuresequenzen durch stringente Waschschritte benötigt wird, führt. Durch das beschriebene Verfahren wird die Unterscheidung eng verwandter Sequenzen erheblich verbessert.

Gegenstand der Erfindung ist ein Verfahren zum Nachweis von Target-Nukleinsäuren aus einem Gemisch verschiedener Nukleinsäuren, mit den Schritten
A) Bereitstellung einer Oberfläche mit immobilisierten Nukleinsäuren oder Nukleinsäure-Analoga, die geeignet sind, mit den Target-Nukleinsäuren nicht kovalente (Basenpaar)-Bindungen einzugehen,
B) nicht-stringente Hybridisierung der nachzuweisenden Target- Nukleinsäuren an die immobilisierten Nukleinsäuren aus einer Lösung der Analyt-Nukleinsäure,
C) Markierung der Nukleinsäuren des Analysengemisches mit Markierungselementen, wobei die Markierungselementen ein Molekulargewicht größer 10.000 g/mol besitzen und,
   wobei die Nukleinsäuren an Liganden gekoppelt werden, die an Liganden-bindende Rezeptormoleküle binden, wobei Avidin, Neutravidin oder Streptavidin als Rezeptor und Biotin als Ligand eingesetzt werden,
   und wobei die Markierungseinheiten mit den Rezeptormolekülen verknüpft oder beschichtet sind,
D) einfaches oder mehrfaches Behandeln der Oberfläche mit einer Waschflüssigkeit zur Entfernung schwächer gebundener Nukleinsäuren unter stringenten Bedingungen mit temperierten Puffer-Lösungen, wobei die Temperatur der Puffer-Lösung mehr als 60° C beträgt, und
E) Nachweis der an der Oberfläche verbliebenen Nukleinsäurepaare anhand der mit ihnen verbundenen Markierungseinheit mittels optischer, optisch spektroskopischer, elektrischer, mechanischer oder magnetischer Nachweismethoden,
wobei der Schritt C) auch vor dem Schritt B) durchgeführt werden kann.

Stringente Waschschritte können bevorzugt durch Waschen des DNA-Arrays mit temperierten Puffer-Lösungen ausgeführt werden, wobei die Temperatur der Puffer-Lösungen oberhalb der für die Hybridisierung der Analyt-Nukleinsäure an die immobilisierten Nukleinsäuren verwendeten Temperatur liegt.

Dem Fachmann ist bekannt, dass die Temperatur, bei der Streptavidin zur Hälfte denaturiert vorliegt, bei 75°C liegt. Bei Sättigung aller Bindungstaschen von Streptavidin durch Biotin kommt es zu einer signifikanten Stabilisierung gegenüber thermisch-induzierter Denaturierung (M. Gonzalez et al. Biomol. Eng. 16, 67-72 (1999)). Werden Goldpartikel mit Streptavidin beschichtet und anschließend an biotinylierte Targets gebunden, so kommt es in der Regel nicht zu einer Sättigung der Streptavidin-Moleküle mit Biotin. Der Fachmann geht deshalb davon aus, dass Streptavidin-beschichtete Goldpartikel nicht stabil sind gegenüber Temperaturen oberhalb von 60-70°C, sondern die Denaturierung des Proteins zu einer Koagulation der Goldpartikel führt. Der Fachmann wird es gemäß dem Stand der Technik entsprechend vermeiden, nach der Markierung von an Arrays hybridisierten Nukleinsäuren mit z.B. Streptavidin-beschichteten Goldpartikeln, Waschschritte bei Temperaturen oberhalb von 60-70°C auszuführen.

Die vorliegende Erfindung beruht auf der Beobachtung, dass z.B. Streptavidin-beschichtete Goldpartikel über eine ausreichende thermische Stabilität verfügen, um stringente Waschschritte auf Arrays nach der Markierung der hybridisierten Nukleinsäuren durchzuführen.

Stringente Waschschritte können bevorzugt auch durch Waschen des DNA-Arrays mit Puffer-Lösungen ausgeführt werden, deren Ionenstärke unterhalb der Ionenstärke der für die Hybridisierung an die immobilisierten Nukleinsäuren verwendeten Pufferlösung liegt. Erfindungsgemäß können beide Verfahren zur Einstellung der Stringenz auch beliebig kombiniert werden.

Eine bevorzugte Alternative des Verfahrens ist dadurch gekennzeichnet, dass die stringenten Waschschritte mit Natriumchlorid-Puffer-Lösungen durchgeführt werden, wobei die Konzentration der Puffer-Lösung unterhalb der für die Hybridisierung an die immobilisierten Nukleinsäuren gewählten Natriumchlorid-Konzentration liegt.

Die Markierung der Target-Nukleinsäuren erfolgt mit Markierungseinheiten. Die Bindung zwischen Nukleinsäure und Markierungseinheit kann durch kovalente Bindungen, koordinative Bindungen, oder nichtkovalente Bindungen erfolgen. Zur Anbindung der Markierungseinheiten muss die Target-Nukleinsäure mit Ligand-Molekülen funktionalisiert werden, die wiederum an Liganden-bindende Rezeptormoleküle binden, mit denen die Oberfläche der Markierungseinheiten beschichtet wurde. Geeignete Rezeptor-Ligand Paare sind dem Fachmann bekannt. Beispiele für Rezeptor-Ligand-Paare sind Biotin-Streptavidin oder Antikörper-Antigen.

Bevorzugt wird als Rezeptor-Ligand-Wechselwirkung eine Wechselwirkung zwischen Avidin, Neutravidin oder Streptavidin als Rezeptor und Biotin als Ligand gewählt.

Alternativ wird die Rezeptor-Ligand-Wechselwirkung bevorzugt eine Wechselwirkung zwischen einem Antikörper und seinem Antigen als Ligand sein.

Die Verknüpfung der Target-Nukleinsäure mit Liganden erfolgt besonders bevorzugt durch enzymatische oder chemische Verfahren oder durch Interkalation.

Die Funktionalisierung der Nukleinsäuren mit Liganden erfolgt durch dem Fachmann bekannte Verfahren. Beispiele für geeignete Funktionalisierungen sind die Verwendung modifizierter Nukleotide in enzymatischen Reaktionen wie PCR, Primer-Extensionen, Transkriptionsreaktionen oder die Verwendung Liganden-gekoppelter Primer in enzymatischen Reaktionen wie PCR oder Primer-Extensionsreaktionen. Die Ankopplung von Liganden an Nukleinsäuren kann beispielsweise auch durch interkalierende Moleküle sowie (photo)chemische Reaktionen zwischen der Nukleinsäure und geeigneten Liganden erfolgen.

Die Markierungseinheiten werden durch Kopplung an Rezeptor-Moleküle modifiziert, derart, dass eine Anbindung an die Liganden möglich ist, mit denen die nachzuweisende Nukleinsäure verknüpft wurde. Beispiele für solche Kopplungen sind die Beschichtung von kolloidalen Goldpartikeln mit Streptavidin oder Antikörpern.

Die Markierungseinheiten sind so ausgewählt, dass sie über optische, optisch spektroskopische, elektrische, mechanische oder magnetische Verfahren ausgelesen werden können. Weiterhin verfügen die Markierungseinheiten über Eigenschaften, die den Verlauf der Ablösung der an den DNA-Array hybridisierten Target-Nukleinsäuren derart verändern, dass die zur Ablösung der markierten Target-Nukleinsäure benötigte Stringenz (hinsichtlich z.B. Temperatur, Ionenstärke) höher ist, als die zur Ablösung einer nichtmarkierten Target-Nukleinsäure benötigte Stringenz.

Als Markierungseinheiten können bevorzugt Nanopartikel, Metallkomplexe und/oder Cluster aus Materialien wie Au, Ag, Pt, Pd, Cu, C usw. eingesetzt werden. Weitere bevorzugte Beispiele für Markierungseinheiten sind Beads, metallbeschichtete Beads, Kohlenstoff-Nanoröhren, Proteine oder andere Moleküle, mit einem Molekulargewicht von bevorzugt >10.000 g/mol und einer Partikelgröße von bevorzugt 1 nm bis ca. 10 µm. Das Molekulargewicht wird dabei bei molekularen Verbindungen aus der Summenformel, bei Polymeren oder metallischen Partikeln nach der Lichtstreuungsmethode als Zahlengemitteltes Molgewicht Mn bestimmt.

Bevorzugt ist auch ein Verfahren das dadurch gekennzeichnet ist, dass die Oberfläche einen Satz aus unterschiedlichen immobilisierten Nukleinsäuren oder Nukleinsäure-Analoga aufweist.

Die mit den Markierungseinheiten markierten DNA-Arrays können durch optische, elektrische, mechanische oder magnetische Verfahren ausgelesen werden.

Besonders bevorzugt ist ein Verfahren bei dem die nachzuweisende Nukleinsäure mit Biotin als Ligand verknüpft wird und die Markierung durch mit Avidin, Neutravidin oder Streptavidin als Rezeptor beschichtete Goldpartikel erfolgt.

Eine Variante, bei der die nachzuweisende Nukleinsäure mit einem Antigen verknüpft wird und die Markierung durch mit Antikörpern beschichtete Goldpartikel erfolgt, wird auch beschrieben.

Die Markierungseinheiten können vor oder während des Auslesens (Schritt E) verstärkt werden. Eine geeignete Verstärkungsreaktion ist z.B. die autometallographische Verstärkung von Metallkolloiden mit beispielsweise Au- oder Ag-basierten Verstärkungslösungen.

Gegenstand der Erfindung ist ferner die Verwendung des erfindungsgemäßen Verfahrens zur Expressions-Profilierung von Ribonukleinsäuren, oder zur Analyse von Einzel-Punktmutationen (SNPs) und zur Analyse amplifizierter Gene:
- Expression Profiling: Nichtstringente Hybridisierung der zu analysierenden RNA-Mischung oder daraus durch enzymatische Reaktionen hergestellten DNA-Mischung (z.B. cDNA). Verknüpfung der RNA- oder DNA-Mischung mit Markierungseinheiten vor der Durchführung stringenter Waschschritte.
- SNPs: Nichtstringente Hybridisierung der die SNPs enthaltenden DNA Mischung oder daraus durch enzymatische Reaktionen hergestellten DNA-Mischung. Verknüpfung der DNA-Mischung mit Markierungseinheiten vor der nichtstringenten Hybridisierung
- Nachweis amplifizierter Gene: analog zu B)

Das erfindungsgemäße Verfahren besitzt gegenüber den dem Stand der Technik nach bekannten Verfahren zum Nachweis von Nukleinsäuren auf DNA-Arrays durch Markierungselemente folgende Vorteile:
- Die Unterscheidung eng verwandter Nukleinsäuren durch Hybridisierungen wird verbessert, wobei die bei einer nicht stringenten Hybridisierung erreichte Signalintensität für die zu selektierende Hybridisierungsreaktion erhalten bleibt.
- Die Verknüpfung der Target-Nukleinsäure kann vor oder nach der nicht stringenten Hybridisierung erfolgen.
- Die Kopplung der Markierungseinheiten an die Target-Nukleinsäure erfolgt nicht durch Hybridisierung und ist somit unabhängig von der Sequenz des Targets.
- Dem Fachmann ist eine große Auswahl verschiedener Markierungseinheiten bekannt, die zur Markierung verwendet werden können. Dies ermöglicht das Auslesen von DNA-Arrays durch verschiedene Verfahren.

Im folgenden wird die Erfindung anhand von Ausführungsbeispielen näher erläutert.

### Beispiele

### Beispiel 1: Vergleich der Diskriminierung vor und nach der Markierung

Es wurden 2 DNA-Chips hergestellt, indem 5'-Amino-modifizierte, Allel-spezifische Oligonukleotide kovalent an oxidierte Silizium-Chips, die zuvor mit amingruppenhaltigen Polymeren beschichtet wurden, immobilisiert wurden. Die kovalente Immobilisierung erfolgte mittels des homobifunktionellen Crosslinkers BS3 (Bis-Sulfo-succinimidyl-suberat, Firma Pierce). Die Sequenzen der Allel-spezifischen Oligonukleotide waren: 5'-Amino- ttt ttt ttt cct aac tcg aac cc (C-Probe) und 5'-Amino- ttt ttt ttt cct aac ttg aac cc (T-Probe). Die Chips hatten eine Größe von 1 cm². Die Allel-spezifischen Oligonukleotide wurden in Duplikaten á 5 µl auf der Chipoberfläche immobilisiert, sodass 4 Spots pro Chip erhalten wurden.

Die DNA eines Patienten, der den CETP (Cholesteryl Ester Transferase Protein Gen)-TaqIB-Genotyp AA besaß, wurde durch PCR nach Standardverfahren amplifiziert, wobei ein biotinylierter und ein nicht biotinylierter Primer verwendet wurde. Der Biotin-Primer besaß die Sequenz 5'-Biotin - ttg tgt ttg tct gcg acc, die Sequenz des nicht biotinylierten Primers war 5'-ccc aac acc aaa tat aca cca.

Das biotinylierte Strang des PCR-Produktes besaß die Sequenz:

5'-Biotin- tt gtgtttgtct gcgacccaga atcactgggg ttcAagttag ggttcagatc tgagccaggt tagggggtta atgtcagggg gtaaagatta ggaggttggt gtatatttgg tgttggg; A=SNP Position

J2x 50 µl des PCR Produktes wurden durch Zentrifugation in Microcon-3-Säulen (Firma Millipore, MWCO=3.000) entsalzt. Die entsalzten PCR-Produkte wurden in 45 µl 0,11N NaOH, 0,9M NaCl, 0,005% SDS gelöst. Die alkalischen Target-Lösungen wurden auf die zwei identisch präparierten DNA-Chips appliziert und 10 min bei 25°C inkubiert.

Es folgte die Neutralisation der alkalischen Hybridisierungslösung durch Zugabe von 5 µl 1M NaH₂PO₄, 1M NaCl, 0,005% SDS. Die Chips mit den Hybridisierungslösungen wurden 15h bei 25°C in einer feuchten Kammer inkubiert (= nicht stringente Hybridisierung).

100 µl einer Lösung von Streptavidin-Gold (10 nm, Sigma) wurden 30 min bei 21.000 g zentrifugiert. Der Überstand wurde entfernt und der Rückstand in 0,1M Phosphatpuffer (pH 8,2), 1M NaCl, 0,005% SDS (= Puffer A) aufgenommen.

Nach Beendigung der nicht stringenten Hybridisierungen wurden die Chips mit Puffer A gewaschen und anschließend bei 25°C getrocknet.

Chip 1: Chip 1 wurde 2h mit 50 µl der Streptavidin-Gold-Lösung bei 25°C inkubiert. Es folgte die Diskriminierung der Allele unter stringenten Bedingungen. Die Diskriminierung erfolgte durch Waschen des Chips für 5 min mit vorgewärmtem Puffer A bei 75°C. Man entfernte Puffer A und wusch mit 0,1M Phosphatpuffer (pH 8,2), 1M NaNO₃, 0,005% SDS um vor der Silberverstärkung störende Chlorid-Ionen zu entfernen.

Chip 2: Chip 2 wurde 5 min mit vorgewärmtem Puffer A bei 55°C gewaschen. Anschließend wurde 2h mit 50 µl der Streptavidin-Gold-Lösung bei 25°C inkubiert. Vor der Silber-Verstärkung wusch man bei 25°C mit 0,1M Phosphatpuffer (pH 8,2), 1M NaNO3, 0,005% SDS.

Zur Silberverstärkung beider Chips bereitete man eine Lösung durch Mischen eines Teils einer wässrigen 0,012M AgNO₃ Lösung sowie vier Teilen einer wässrigen Lösung von 0,05M Hydrochinon und 0,3M Natriumcitratpuffer (pH 3,8). Die Chips wurden 30 min in diese Lösung eingetaucht. Die Silberverstärkung wurde durch Waschen der Chips mit Wasser gestoppt.

Die Gleichstrom-Widerstandsmessung der Silber-verstärkten Chip-Oberflächen erfolgte zwischen extern aufgesetzten Elektroden. Um Inhomogenitäten auf der Probe auszumitteln wurden pro DNA-Spot 25 Messungen an verschiedenen Stellen des Spots mit einer automatisierten Messapparatur durchgeführt. Der Kern dieser Apparatur besteht aus einem Probentisch und zwei metallischen Messspitzen. Sowohl Probentisch als auch Messspitzen werden unter Computerkontrolle bewegt. Zur elektrischen Charakterisierung wird der Tisch schrittweise in einem rechteckförmigen Raster bewegt. An jedem Rasterpunkt werden die beiden Messspitzen abgesenkt, sodass sie einen elektrischen Kontakt mit der Probe ausbilden. Die Gleichstromwiderstandsmessung erfolgt in einer Zweipunktanordnung mit einem Multimeter (Multimeter 2000, Keithley Instruments), dessen Eingänge mit den beiden Messspitzen verbunden sind. Das jeweilige Messresultat wird bei Unterschreiten von 1 MOhm als positiv leitfähig eingestuft bzw. bei Überschreiten von 1MOhm als negativ bewertet. Der Quotient aus der Anzahl positiver Messungen und Gesamtzahl von Messungen definiert die normierte Leitfähigkeit als die zu betrachtende Messgröße.

Die Ergebnisse der Gleichstrom-Widerstandsmessungen sind in untenstehender Tabelle 1 zusammengefasst:

| | **norm. Leitfähigkeit [%]** | **norm. Leitfähigkeit [%]** |
|---|---|---|
| **Chip1** | **Spot 1** | **Spot 2** |
| T-Probe | 100 | 100 |
| C-Probe | 0 | 0 |

| **Chip 2** | | |
|---|---|---|
| T-Probe | 0 | 0 |
| C-Probe | 0 | 0 |

Die Ergebnisse demonstrieren, dass durch die Markierung der Target-Nukleinsäure vor der Diskriminierung (Chip 1) eine sehr gute Unterscheidung zwischen den Allelen erreicht wird. Zudem wird das absolute Signal auf dem "match"-Spot vergrößert, was unter den gewählten Bedingungen den elektrischen Nachweis der Nukleinsäure-Hybridisierung überhaupt erst ermöglicht. Dementsprechend ist auf Chip 2 weder für das eine noch das andere Allel ein Signal zu detektieren.

### Beispiel 2: Analyse der PCR-Produkte von Patientenproben

Die DNA 19 verschiedener Patienten wurde mittels dem Stand der Technik entsprechenden Genotypisierungsverfahren (z.B. Pyrosequencing) auf ihren CETP-Taq1-Genotyp hin untersucht. Anschließend wurde die DNA der 19 verschiedenen Patienten durch PCR nach Standardverfahren amplifiziert, wobei ein biotinylierter und ein nicht biotinylierter Primer verwendet wurde. Der Biotin-Primer besaß die Sequenz 5'-Biotin - ttg tgt ttg tct gcg acc, die Sequenz des nicht biotinylierten Primers war 5'-ccc aac acc aaa tat aca cca.

Das biotinylierte Strang des PCR-Produktes besaß die Sequenz:

5'-Biotin- tt gtgtttgtct gcgacccaga atcactgggg ttcRagttag ggttcagatc tgagccaggt tagggggtta atgtcagggg gtaaagatta ggaggttggt gtatatttgg tgttggg; R= A oder G

Es wurden 19 DNA-Chips hergestellt, indem 5'-Amino-modifizierte, Allel-spezifische Oligonukleotide kovalent an oxidierte Silizium-Chips, die mit amingruppenhaltigen Polymeren beschichtet waren, immobilisiert wurden. Die kovalente Immobilisierung erfolgte mittels des homobifunktionellen Crosslinkers BS3 (Bis-Sulfo-succinimidyl-suberat, Firma Pierce). Die Sequenzen der Allel-spezifischen Oligonukleotide waren: 5'-Amino- ttt ttt ttt cct aac tcg aac cc (spezifisch für G-Allel) und 5'-Amino- ttt ttt ttt cct aac ttg aac cc (spezifisch für A-Allel). Die Chips hatten eine Größe von 1cm2. Die Allel-spezifischen Oligonukleotide wurden in Duplikaten á 5µl auf der Chipoberfläche immobilisiert, sodass 4 Spots pro Chip erhalten wurden.

Jeweils 50 µl der PCR Produkte wurden durch Zentrifugation in Microcon-3-Säulen (Firma Millipore, MWCO=3.000) entsalzt. Die entsalzten PCR-Produkte wurden in 45 µl 0,11N NaOH, 0,9M NaCl, 0,005% SDS gelöst. Die alkalische Target-Lösungen wurde auf die 19 DNA-Chips appliziert und 10 min bei 25°C inkubiert.

Es folgte die Neutralisation der alkalischen Hybridisierungslösung durch Zugabe von 5µl 1M NaH₂PO₄, 1M NaCl, 0,005% SDS. Die Chips mit den Hybridisierungslösungen wurde 15h bei 25°C in einer feuchten Kammer inkubiert (= nicht stringente Hybridisierung).

19 x 50 µl einer Lösung von Streptavidin-Gold (10 nm, Sigma) wurden 30 min bei 21.000 g zentrifugiert. Die Überstände wurden entfernt und die Rückstände in 0,1M Phosphatpuffer (pH 8,2), 1M NaCl, 0,005% SDS (= Puffer A) aufgenommen.

Nach Beendigung der nicht stringenten Hybridisierungen wurden die Chips mit Puffer A gewaschen und anschließend bei 25°C getrocknet. Die Chips wurden 2h mit je 50 µl der Streptavidin-Gold-Lösung bei 25°C inkubiert. Es folgte die Diskriminierung der Allele unter stringenten Bedingungen. Die Diskriminierung erfolgte durch Waschen der Chips für 5 min mit vorgewärmtem Puffer A bei 60°C. Man entfernte Puffer A und wusch mit 0,1M Phosphatpuffer (pH 8,2), 1M NaNO₃, 0,005% SDS um vor der Silberverstärkung störende Chlorid-Ionen zu entfernen.

Zur Silberverstärkung bereitete man eine Lösung durch Mischen eines Teils einer wässrigen 0,012M AgNO₃ Lösung sowie vier Teilen einer wässrigen Lösung von 0,05M Hydrochinon und 0,3M Natriumcitratpuffer (pH 3,8). Die Chips wurden 9 min in diese Lösung eingetaucht. Die Silberverstärkung wurde durch Waschen der Chips mit Wasser gestoppt.

Die Gleichstrom-Widerstandsmessung der Silber-verstärkten Chip-Oberflächen erfolgte zwischen extern aufgesetzte Elektroden. Analog zu Beispiel 1 wurden pro DNA-Spot 25 Messungen an verschiedenen Stellen des Spots durchgeführt.

Die Ergebnisse der Gleichstromwiderstandsmessungen der für 9 min Silber-verstärkten Chips sind in Tabelle 2 dargestellt. Diejenigen Chips, die nach 9 min Silberverstärkung noch keine Signale zeigten, wurden weitere 4 min mit der frisch hergestellten Silbernitrat/Hydrochinon-Lösung behandelt. Nach Abbruch der Verstärkungsreaktion durch Waschen mit Wasser erfolgte die Gleichstrom-Widerstandsmessung nach dem oben dargestellten Verfahren.

Die Ergebnisse sind in Tabelle 2 dargestellt. 18 der 19 Genotypen wurden korrekt bestimmt. In einem Fall (Patient 13) wurde ein homozygot G-Allel-tragender Patient als heterozygoter Patient bestimmt.

**Tabelle 2: Ergebnis der Analyse der PCR-Produkte von Patientenproben**

| Patient | T-Spot, 9' (norm.Leitfähigkeit [%]) | T-Spot, 13' (norm.Leitfähigkeit [%]) | C-Spot, 9' (norm.Leitfähigkeit [%]) | C-Spot, 13' (norm.Leitfähigkeit [%]) | Referenz-Genotyp |
|---|---|---|---|---|---|
| 16 | 100 | nicht gemessen | 0 | nicht gemessen | AA |
| 13 | 100 | nicht gemessen | 100 | nicht gemessen | GG |
| 5 | 0 | nicht gemessen | 20 | nicht gemessen | GG |
| 14 | 60 | nicht gemessen | 0 | nicht gemessen | AA |
| 12 | 100 | nicht gemessen. | 0 | nicht gemessen | AA |
| 3 | 0 | 90 | 0 | 70 | AG |
| 6 | 0 | 100 | 0 | 50 | AG |
| 4 | 0 | 80 | 0 | 100 | AG |
| 2 | 0 | 35 | 0 | 50 | AG |
| 47 | 10 | nicht gemessen | 100 | nicht gemessen | GG |
| 45 | 10 | nicht gemessen | 100 | nicht gemessen | GG |
| 35 | 20 | nicht gemessen | 100 | nicht gemessen | GG |
| 32 | 0 | nicht gemessen | 100 | nicht gemessen | GG |
| 46 | 100 | nicht gemessen | 0 | nicht gemessen | AA |
| 44 | 100 | nicht gemessen | 0 | nicht gemessen | AA |
| 49 | 0 | 100 | 0 | 100 | AG |
| 50 | 0 | 100 | 0 | 100 | AG |
| 42 | 0 | 100 | 0 | 100 | AG |
| 41 | 0 | 100 | 0 | 100 | AG |

## Patentansprüche

1. Verfahren zum Nachweis von Target-Nukleinsäuren aus einem Gemisch verschiedener Nukleinsäuren, mit den Schritten
A) Bereitstellung einer Oberfläche mit immobilisierten Nukleinsäuren oder Nukleinsäure-Analoga, die geeignet sind, mit den Target-Nukleinsäuren nicht kovalente (Basenpaar)-Bindungen einzugehen,
B) nicht-stringente Hybridisierung der nachzuweisenden Target- Nukleinsäuren an die immobilisierten Nukleinsäuren aus einer Lösung der Analyt-Nukleinsäure,
C) Markierung der Nukleinsäuren des Analysengemisches mit Markierungselementen, wobei die Markierungselementen ein Molekulargewicht größer 10.000 g/mol besitzen und,
wobei die Nukleinsäuren an Liganden gekoppelt werden, die an Liganden-bindende Rezeptormoleküle binden, wobei Avidin, Neutravidin oder Streptavidin als Rezeptor und Biotin als Ligand eingesetzt werden,
und wobei die Markierungseinheiten mit den Rezeptormolekülen verknüpft oder beschichtet sind,
D) einfaches oder mehrfaches Behandeln der Oberfläche mit einer Waschflüssigkeit zur Entfernung schwächer gebundener Nukleinsäuren unter stringenten Bedingungen mit temperierten Puffer-Lösungen, wobei die Temperatur der Puffer-Lösung mehr als 60° C beträgt, und
E) Nachweis der an der Oberfläche verbliebenen Nukleinsäurepaare anhand der mit ihnen verbundenen Markierungseinheit mittels optischer, optisch spektroskopischer, elektrischer, mechanischer oder magnetischer Nachweismethoden,
wobei der Schritt C) auch vor dem Schritt B) durchgeführt werden kann.

2. Verfahren nach Anspruch 1, **dadurch gekennzeichnet, dass** die Temperatur der Puffer-Lösung in Schritt D) 75° C beträgt.

3. Verfahren nach Anspruch 1 oder 2, **dadurch gekennzeichnet, dass** Schritt D) unter stringenten Bedingungen mit temperierten Puffer-Lösungen durchgeführt wird, wobei die Ionenstärke der Puffer-Lösung unterhalb der für die Hybridisierung der Analyt-Nukleinsäure an die immobilisierten Nukleinsäuren gewählten Ionenstärke der Analytlösung liegt.

4. Verfahren nach Anspruch 3, **dadurch gekennzeichnet, dass** Schritt D) unter stringenten Bedingungen mit Natriumchlorid-Puffer-Lösungen durchgeführt wird, wobei die Konzentration der Puffer-Lösung unterhalb der für die Hybridisierung an die immobilisierten Nukleinsäuren gewählten Natirumchlorid-Konzentration liegt.

5. Verfahren nach Anspruch 1 bis 4, **dadurch gekennzeichnet, dass** die Verknüpfung der Target-Nukleinsäure mit Liganden durch enzymatische oder chemische Verfahren oder durch Interkalation erfolgt.

6. Verfahren nach einem der Ansprüche 1 bis 5, **dadurch gekennzeichnet, dass** die Markierungseinheiten Nanopartikel, Metallkomplexe und/oder Cluster auf Basis der Elemente der Reihe Au, Ag, Pt, Pd und C sind.

7. Verfahren nach einem der Ansprüche 1 bis 6, **dadurch gekennzeichnet, dass** die Markierungseinheiten vor oder während des Nachweises E) verstärkt werden.

8. Verfahren nach einem der Ansprüche 1 bis 7, **dadurch gekennzeichnet, dass** die Oberfläche einen Satz aus unterschiedlichen immobilisierten Nukleinsäuren oder Nukleinsäure-Analoga aufweist.

9. Verfahren nach einem der Ansprüche 1 bis 8, **dadurch gekennzeichnet, dass** die nachzuweisende Nukleinsäure mit Biotin als Ligand verknüpft wird und die Markierung durch mit Avidin, Neutravidin oder Streptavidin als Rezeptor beschichtete Goldpartikel erfolgt.

10. Verfahren nach Anspruch 8, **dadurch gekennzeichnet, dass** die Verstärkung der Goldpartikel durch eine autometallographische Reaktion erfolgt. und der Nachweis der Nukleinsäure optisch oder elektrisch erfolgt.

11. Verwendung des Verfahrens nach einem der Ansprüche 1 bis 10 zur Expressions-Profilierung von Ribonukleinsäuren, oder zur Analyse von Einzel-Punktmutationen (SNPs) und zur Analyse amplifizierter Gene.

## Claims

1. Method for the detection of target nucleic acids from a mixture of different nucleic acids, with the steps of
A) providing a surface having immobilized nucleic acids or nucleic-acid analogues, which are suitable for forming non-covalent (base-pair) bonds with the target nucleic acids,
B) non-stringent hybridization of the target nucleic acids to be detected onto the immobilized nucleic acids from a solution of the analyte nucleic acid,
C) labelling of the nucleic acids of the analysis mixture with labelling elements,
where the labelling elements have a molecular weight of more than 10,000 g/mol and,
where the nucleic acids are coupled to ligands which bind to ligand-binding receptor molecules, where avidin, neutravidin or streptavidin are used as the receptor and biotin as the ligand,
and where the labelling units are linked or coated with the receptor molecules,
D) single or repeated treatment of the surface with a washing liquid in order to remove weakly bound nucleic acids under stringent conditions with thermally regulated buffer solutions, the temperature of the buffer solution being greater than 60°C, and
E) detection of the nucleic-acid pairs remaining on the surface with the aid of the labelling unit bonded to them, by means of optical, optical-spectroscopic, electrical, mechanical or magnetic detection methods,
where step C) can also be carried out before step B).

2. Method according to Claim 1, **characterized in that** the temperature of the buffer solution in step D) is 75°C.

3. Method according to Claim 1 or 2, **characterized in that** step D) is carried out under stringent conditions with thermally regulated buffer solutions, the ionic strength of the buffer solution lying below the analyte-solution ionic strength selected for the hybridization of the analyte nucleic acid onto the immobilized nucleic acids.

4. Method according to Claim 3, **characterized in that** step D) is carried out under stringent conditions with sodium-chloride buffer solutions, the concentration of the buffer solution lying below the sodium-chloride concentration selected for the hybridization onto the immobilized nucleic acids.

5. Method according to Claims 1 to 4, **characterized in that** the linking of the target nucleic acid with ligands is carried out by enzymatic or chemical methods or by intercalation.

6. Method according to one of Claims 1 to 5, **characterized in that** the labelling units are nanoparticles, metal complexes and/or clusters based on elements from the list Au, Ag, Pt, Pd and C.

7. Method according to one of Claims 1 to 6, **characterized in that** the labelling units are enhanced before or during the detection E).

8. Method according to one of Claims 1 to 7, **characterized in that** the surface has a set of different immobilized nucleic acids or nucleic-acid analogues.

9. Method according to one of Claims 1 to 8, **characterized in that** the nucleic acid to be detected is linked with biotin as a ligand, and the labelling is carried out using gold particles coated with avidin, neutravidin or streptavidin as a receptor.

10. Method according to Claim 8, **characterized in that** the gold particles are enhanced by an autometallographic reaction. and the detection of the nucleic acid is carried out optically or electrically.

11. Use of the method according to one of Claims 1 to 10 for the expression profiling of ribonucleic acids, or for the analysis of single nucleotide polymorphisms (SNPs) and for the analysis of amplified genes.

## Revendications

1. Procédé pour la détection d'acides nucléiques cibles à partir d'un mélange d'acides nucléiques différents, comprenant les étapes de
A) Préparation d'une surface avec des acides nucléiques ou des analogues d'acide nucléique immobilisés, qui sont appropriés pour s'engager dans des liaisons non covalentes (paires de bases) avec les acides nucléiques cibles,
B) Hybridation non rigoureuse des acides nucléiques cibles avec les acides nucléiques immobilisés à partir d'une solution d'acides nucléiques analytes,
C) Marquage des acides nucléiques du mélange d'analyse avec des éléments de marquage, où les éléments de marquage possèdent un poids moléculaire supérieur à 10 000 g/mole et
où les acides nucléiques sont couplés à des ligands, qui se lient à des molécules récepteurs liant les ligands, où l'avidine, la neutravidine ou la streptavidine sont mis en oeuvre comme récepteur et la biotine comme ligand, et
où les unités de marquage sont reliées ou recouvertes des molécules récepteurs,
D) Traitement simple ou multiple de la surface avec un liquide de lavage pour éliminer les acides nucléiques faiblement liés, dans des conditions rigoureuses avec des solutions tampons tempérées, où la température de la solution tampon est supérieure à 60°C, et
E) Détection des paires d'acides nucléiques restant sur la surface, a l'aide de l'unité de marquage y reliée, au moyen de procédés de détection optiques, optico-spectroscopiques, électriques, mécaniques ou magnétiques,
où l'étape C) peut également être réalisée avant l'étape B).

2. Procédé selon la revendication 1, **caractérisé en ce que** la température de la solution tampon à l'étape D) s'élève à 75°C.

3. Procédé selon la revendication 1 ou 2, **caractérisé en ce que** l'étape D) est réalisée dans des conditions rigoureuses avec des solutions tampons tempérées, où la force ionique de la solution tampon se situe sous la force ionique choisie pour l'hybridation des acides nucléiques analytes avec les acides nucléiques immobilisés.

4. Procédé selon la revendication 3, **caractérisé en ce que** l'étape D) est réalisée dans des conditions rigoureuses avec des solutions tampons au chlorure de sodium, où la concentration de la solution tampon se situe sous la concentration en chlorure de sodium choisie pour l'hybridation avec les acides nucléiques immobilisés.

5. Procédé selon les revendications 1 à 4, **caractérisé en ce que** le lien des acides nucléiques cibles aux ligands est réalisé par un procédé enzymatique ou chimique ou par intercalation.

6. Procédé selon l'une des revendications 1 à 5, **caractérisé en ce que** les unités de marquage sont des nanoparticules, des complexes et/ou clusters métalliques à base des éléments de la série Au, Ag, Pt, Pd et C.

7. Procédé selon l'une des revendications 1 à 6, **caractérisé en ce que** les unités de marquage sont renforcées avant ou pendant la détection E).

8. Procédé selon l'une des revendications 1 à 7, **caractérisé en ce que** la surface présente un jeu de différents acides nucléiques ou analogues d'acide nucléique immobilisés.

9. Procédé selon l'une des revendications 1 à 8, **caractérisé en ce que** l'acide nucléique à détecter est relié à la biotine comme ligand et le marquage est réalisé par des particules d'or revêtues d"avidine, de neutravidine ou de streptavidine comme récepteur.

10. Procédé selon la revendication 8, **caractérisé en ce que** le renforcement des particules d'or est réalisé par une réaction auto-métallographique et **en ce que** la détection de l'acide nucléique est réalisée de manière optique ou électrique.

11. Utilisation du procédé selon les revendications 1 à 10, pour établir le profil d'expression d'acides ribonucléiques, ou pour l'analyse de mutations ponctuelles (SNP) et pour l'analyse de gènes amplifiés.
